# EUROPEAN PATENT APPLICATION

(11) **EP 2 184 053 A1**
(43) Date of publication of application: **12.05.2010**
(21) Application number: 08805344.2
(22) Date of filing: 24.07.2008
(51) Int. Cl.: A61K 8/97, A61K 8/92

(54) **METHOD FOR OBTAINING A COSMETIC PRODUCT FROM A CONCENTRATE OF THE TIGER NUT DRINK**

(30) Priority: 24.07.2007 ES 200702058
(71) Applicant: Horchateria Daniel, S.l., 46120 Alboraya Valencia (ES)
(72) Inventor: TORTAJADA FORNER, Daniel, E-46120 Alboraya (Valencia) (ES); NOVEJARQUE CONDE,José Antonio, E-46120 Alboraya (Valencia) (ES)
(74) Representative: Ungria Lopez, Javier
(86) International application number: PCT/ES2008/000515
(87) International publication number: WO 2009/013377

(57) **Abstract**

The present invention relates to a method for obtaining a cosmetic product from a tiger nut milk concentrate, **characterized in that** it comprises:
a) mixing in a tank sugar-free tiger nut milk with cosmetic ingredients and thermal deionized distilled water;
b) mixing in a second tank surfactants with molasses alcohol;
c) mixing the products resulting from the previous steps in order to produce a base concentrate;
d) applying ultrasounds to said base concentrate; and
e) adding pharmaceutically acceptable excipients,

as well as to the base concentrate, and to the cosmetic product resulting from said method.

## Description

### TECHNICAL FIELD OF THE INVENTION

The present invention is included in the field of the preparation of cosmetic products, more specifically, cosmetic products based on tiger nut milk concentrate.

### STATE OF THE ART PRIOR TO THE INVENTION

Tiger nut is planted on the months of April and May. The harvest is carried out between the months of November and January.

Once the harvesting process is finished, a wash thereof for cleaning and impurities removal is carried out. Next, a deep cleaning process for undesirable elements (straw and stones from the country) is applied. Subsequently the down is removed and a fine water shower for finishing the cleaning is provided.

Once the tiger nut is clean, the moisture level is then reduced by drying it. In this process is necessary to achieve a moisture reduction from 50% to 15%. Said drying time lasts about three months and is carried out in drying chambers in a slow and careful manner.

Once it is dried, its cleaning and sorting is then carried out in order to separate the impurities, the faulty or those of small size.

Once this cleaning and sorting operation has been carried out, they are packaged in sacs for its use.

From the tiger nuts thus obtained, the natural tiger nut milk is produced, this being a method common with that employed for subsequently preparing the liquid food typical from the Valencia region. This method is comprised essentially of the following steps:
- Tiger nut cleaning and disinfection;
- Grinding of the tiger nut with a hammer mill;
- Water addition;
- Pressing of the tiger nut, separating the pulp from the fluid;
- Obtaining the natural tiger nut milk base which will be employed in the method of the invention.

### DETAILED DESCRIPTION OF THE INVENTION

The present invention relates to a method for obtaining a cosmetic product from a tiger nut milk concentrate, **characterized in that** it comprises:
a) mixing in a tank sugar free-tiger nut milk with cosmetic ingredients and thermal deionized distilled water;
b) mixing in a second tank surfactants with molasses alcohol;
c) mixing the resulting products from the previous steps to produce the base concentrate ;
d) applying ultrasounds to said base concentrate; and
e) adding pharmaceutically acceptable excipients.

The step a) is carried out in a tank which can be made of various materials, but preferably is made of stainless steel.

In a preferred embodiment said method is **characterized in that** said stainless steel tank employed in step a) comprises a propeller mixer with capacity of between 200 and 500 rpm.

In a specific embodiment, said mixture from step a) is carried out at a temperature of between 20 and 70°C. Additionally, preferably, said cosmetic ingredients employed in step a) are selected from: natural cosmetic ingredients, synthetic cosmetic ingredients, and mixtures thereof. These cosmetic ingredients or cosmetic feedstock may be any of those listed in the attachment of the Decision 96/335-CE.

In a specific embodiment, said cosmetic ingredients employed in step a) are selected from combinations of two or more of the following:
- Anciano and Hamamelis distilled water;
- Sclerotium gum;
- water and Cyathea medullaris extract;
- betaine, water, glycerin, hydrogenated Lecithum, honey and pectin;
- Ivy and myrrh extracts, leucine, valine, arginine and lysine;
- glycerin, water, lysolecithin, and seed oil from Perilla frutescens;
- corn and soy unsaponifiable
- propolis in hydroalcoholic solution,
- raspberry seeds virgin vegetable oil;
- rosehip virgin vegetable oil;
- Argan virgin vegetable oil;
- apricot nut virgin vegetable oil;
- tea tree oil.

On the other hand, in a specific embodiment of the method for obtaining a cosmetic product from a tiger nut milk concentrate the tank employed in step b) is selected from a stainless steel tank and a polyester tank. Additionally, in a more preferred embodiment, the surfactants employed in step b) are selected from natural surfactants, synthetic surfactants, and mixtures thereof. In another preferred embodiment said surfactants employed in step b) are selected from anionic, cationic, non-ionic and amphoteric surfactants and mixtures thereof.

In a specific embodiment of the invention, said surfactants employed in step b) are selected from: anionic surfactants such as acyl amino acids (and salts), acyl glutamates, acyl peptides, sarcosinates, taurates, carboxylic acids and their salts, saturated chain acids, carboxylic acids esters, carboxylic acids ethers, phosphoric acid esters (and salts), sulfonic acids (and salts), acyl isotianates, alkyl aryl sulfonates, alkyl sulfonates, sulfosuccinate, sulfuric acid esters; cationic surfactants such as alkyl amines, alkyl imidazolines, ethoxylated amines, alkyl benzyl dimethyl ammonium salts, heterocyclic ammonium salts, tetraalkyl ammonium salts; amphoterics such as acyl amino acids (and derivatives), N-alkyl-amino acids; non-ionic surfactants such as alcohols and alkanolamides, amine oxides, esters.

In a particular embodiment of the method of the invention, the mixture of step c) is carried out during a time comprised between 15 and 25 minutes depending on the product which is intended to obtain, at a pH comprised between 4 and 9.

The mixture of the products obtained in steps a) and b) is carried out in a tank which may be manufactured from various materials and, which is preferably a stainless steel tank

On the other hand, in a particular embodiment of the method of the invention, the mixture of step c) is carried out during 20 minutes at a pH comprised between 4 and 9.

In step d) for ultrasounds application, the ultrasounds application time span will depend on the type of final product to obtain.

Additionally, in a specific embodiment, said step d) for ultrasounds application lasts between 20 and 60 minutes.

The present invention also relates to a tiger nut milk base concentrate for preparing a cosmetic product, **characterized in that** said base concentrate comprises:
- tiger nut milk 100%
- natural or synthetic cosmetic ingredients or mixtures,
- distilled/deionized/thermal water
- natural or synthetic surfactants or mixtures and
- molasses alcohol.

Specific embodiments relate to a tiger nut milk base concentrate for preparing a cosmetic product, wherein said base concentrate comprises:
- tiger nut milk 100%
- natural or synthetic cosmetic ingredients or mixtures,
- distilled/deionized/thermal water
- natural or synthetic surfactants or mixtures and
- molasses alcohol, and
   wherein said cosmetic ingredients are selected from combinations of two or more of the following:
- Anciano and Hamamelis distilled water;
- Sclerotium gum;
- water and Cyathea medullaris extract;
- betaine, water, glycerin, hydrogenated Lecithum, honey and pectin;
- ivy and myrrh extracts, leucine, valine, arginine and lysine;
- glycerin, water, lysolecithin, and seed oil from Perilla frutescens;
- corn and soy unsaponifiable
- propolis in hydroalcoholic solution,
- raspberry seeds virgin vegetable oil;
- rosehip virgin vegetable oil;
- Argan virgin vegetable oil;
- apricot nut virgin vegetable oil;
- tea tree oil.

Generally, in the present invention, the base concentrate is the following:
- concentrate/traditional liquid tiger nut milk (100%);
- thermal/deionized water;
- natural and synthetic surfactants,
- Anciano and Hamamelis distilled water;
- Sclerotium gum;
- water and Cyathea medullaris extract;
- betaine, water, glycerin, hydrogenated Lecithum, honey and pectin;
- ivy and myrrh extracts, leucine, valine, arginine and Lysine;
- glycerin, water, lysolecithin, and seed oil from Perilla frutescens;
- corn and soy unsaponifiable
- propolis in hydroalcoholic solution,
- raspberry seeds virgin vegetable oil;
- rosehip virgin vegetable oil;
- Argan virgin vegetable oil;
- apricot nut virgin vegetable oil;
- tea tree oil.

According to preferred embodiments, said base concentrate has the following composition:
- concentrate/traditional liquid tiger nut milk (100%); 30-50%
- thermal/deionized water; 5-20%
- natural and synthetic surfactants, 1-15%
- Anciano and Hamamelis distilled water; 1-10%
- Sclerotium gum; (Thickener) 1-4%
- water and Cyathea medullaris extract; 1-3%
- betaine, water, glycerin, hydrogenated Lecithum, honey and pectin; (Aqueous Solution of said products) 3-5%
- ivy and myrrh extracts, leucine, valine, arginine and lysine; (essential amino acids) 2-6%
- glycerin, water, lysolecithin, and seed oil from Perilla frutescens; (Aqueous Solution) 1-4%
- corn and soy unsaponifiable 2-5%
- propolis in hydroalcoholic solution, 1-8%
- raspberry seeds virgin vegetable oil;
- rosehip virgin vegetable oil;
- Argan virgin vegetable oil;
- apricot nut virgin vegetable oil;
- Tea tree oil.

According to particular preferred embodiments, the base concentrate contains the mentioned oils:
- raspberry seeds virgin vegetable oil;
- rosehip virgin vegetable oil;
- Argan virgin vegetable oil;
- apricot nut virgin vegetable oil;
- tea tree oil
in combinations at concentrations providing 1-10% in the base concentrate.

According to the invention, the elemental composition of the base concentrate product is the starting point for obtaining the different finished products, which finally compose the cosmetic products and personal hygiene lines.

The present invention also relates to a cosmetic product containing the base concentrate previously defined.

According to particular embodiments, the type formulation of a finished cosmetic product contains:
- natural base concentrate,
- emulsifier,
- active ingredients,
- essential oils,
- antioxidants,
- preservatives,
- natural dyes,
- perfumes, and
- sunscreens and sun protectors.

The products that are obtained from the defined base concentrate and by means of the object method of the present invention are:
- Body cosmetic products: creams, skin lotions,
- Facial cosmetic products
- Color cosmetic products; lipstick, make up, ...
- Personal hygiene products: soaps, bath gels. Deodorants, ...
- Capillary cosmetic products
- Toilet soaps
- Fragrances, perfumes and colognes
- Line of products for sun protection

Finally the desired cosmetic product is obtained with the natural tiger nut special properties and qualities, by checking that the final product follows the next rules:
- Interaction among the ingredients and components themselves (right synergism).
- Interaction of the base concentrate with the remaining ingredients of the final cosmetic products.
- Control of temperature, pH, redox potential and environmental factors (light, air, etc.).
- Interaction of the final product with the vehicle (package).

The present invention further relates to a cosmetic product **characterized in that** it is obtained by the method described in the previous paragraphs and in that it comprises tiger nut milk concentrate.

The tiger nut most important properties and, consequently, those of its tiger nut milk, are due to its oleic acid, starches, essential amino acids, vitamin E, vitamin C, biotin or vitamin H, rutin or vitamin P, minerals such as phosphorous, calcium, magnesium, iron and potassium content.

### MODE OF CARRYING OUT THE INVENTION

Next, an example of specific embodiment of the invention is detailed with the object of illustrating the present invention and in no way limitative thereof.
Generally, the flow chart showed in the following illustrates an embodiment of the method of the invention:
a) mixing in a tank:
   TIGER NUT MILK 100%
   NATURAL/SYNTHETIC COSMETIC INGREDIENTS
   DISTILLED/DEIONIZED/THERMAL WATER
   Stirring at 200-500 r.p.m. between 20-70°C.
b) mixing in a second tank
   NATURAL SURFACTANTS
   SYNTHETIC SURFACTANTS
   MOLASSES ALCOHOL
   Stirring 16 hours at pH 4-9, at 200-500 r.p.m.
c) mixing the products resulting from the previous steps in a stainless steel tank in order to produce a base concentrate and applying ultrasounds.

Starting from the base concentrate previously defined and adding to it different products, a bath gel with the following composition is obtained:
- Base concentrate: 10-15%
- Surfactants: 30-40%
- Amphoterics: Cocamidopropyl Betaine
- Non-ionic: Glicereth-17 Cocoate
- Preservative: 0.1% Usually: Propolis with pine oil
- Natural dye 0.2% of a 1% solution, depending on the color that is intended to obtain, different products,
- Perfume 0.5%. With a fresh note, with spring flowers, combined with wild citric notes and green tea,
- Thickener: Normally is utilized salt (NaCl) 1%,
- Thermal water: selected, diluted with distilled water in order to reduce the concentration of salts: Remainder up to 100%.

## Claims

1. A method for obtaining a cosmetic product from a tiger nut milk concentrate, **characterized in that** it comprises:
a) mixing in a tank sugar-free tiger nut milk with cosmetic ingredients and thermal deionized distilled water;
b) mixing in a second tank surfactants with molasses alcohol;
c) mixing the products resulting from the previous steps in a stainless steel tank in order to produce a base concentrate;
d) applying ultrasounds to said base concentrate; and
f) adding pharmaceutically acceptable excipients.

2. A method for obtaining a cosmetic product from a tiger nut milk concentrate according to claim 1, **characterized in that** said stainless steel tank employed in step a) comprises a propeller mixer with capacity of between 200 and 500 rpm.

3. A method for obtaining a cosmetic product from a tiger nut milk concentrate according to claim 1, **characterized in that** said mixture from step a) is carried out at a temperature between 20 and 70°C.

4. A method for obtaining a cosmetic product from a tiger nut milk concentrate according to claim 1, **characterized in that** said cosmetic ingredients employed in step a) are selected from: natural cosmetic ingredients, synthetic cosmetic ingredients, and mixtures thereof.

5. A method for obtaining a cosmetic product from a tiger nut milk concentrate according to claim 1,
**characterized in that** said cosmetic ingredients employed in step a) are selected from two or more of the following:
- Anciano and Hamamelis distilled water;
- Sclerotium gum;
- water and Cyathea medullaris extract;
- betaine, water, glycerin, hydrogenated Lecithum, honey and pectin;
- ivy and myrrh extracts, leucine, valine, arginine and lysine;
- glycerin, water, lysolecithin, and seed oil from Perilla frutescens;
- corn and soy unsaponifiable
- propolis in hydroalcoholic solution,
- raspberry seeds virgin vegetable oil;
- rosehip virgin vegetable oil;
- Argan virgin vegetable oil;
- apricot nut virgin vegetable oil and
- tea tree oil.

6. A method for obtaining a cosmetic product from a tiger nut milk concentrate according to claim 1, **characterized in that** the tank employed in step b) is selected from a stainless steel tank and a polyester tank.

7. A method for obtaining a cosmetic product from a tiger nut milk concentrate according to claim 1, **characterized in that** the surfactants employed in step b) are selected from natural surfactants, synthetic surfactants, and mixtures thereof.

8. A method for obtaining a cosmetic product from a tiger nut milk concentrate according to claim 1, **characterized in that** the surfactants employed in step b) are selected from anionic, cationic, non-ionic and amphoteric surfactants and mixtures thereof.

9. A method for obtaining a cosmetic product from a tiger nut milk concentrate according to claim 1, **characterized in that** the surfactants employed in step b) are selected from acyl amino acids (and salts), acyl glutamates, acyl peptides, sarcosinates, taurates, carboxylic acids and their salts, saturated chain acids, carboxylic acids esters, carboxylic acids ethers, phosphoric acid esters (and salts), sulfonic acids (and salts), acyl isotianates, alkyl aryl sulfonates, alkyl sulfonates, sulfosuccinate, sulfuric acid esters; alkyl amines, alkyl imidazolines, ethoxylated amines, alkyl benzyl dimethyl ammonium salts, heterocyclic ammonium salts, tetraalkyl ammonium salts; acyl amino acids (and derivatives), N-alkyl-amino acids, alcohols, alcanolamides, amine oxides, esters and combinations thereof.

10. A method for obtaining a cosmetic product from a tiger nut milk concentrate according to claim 1, **characterized in that** the mixture from step c) is carried out during a time comprised between 15 and 25 minutes at a pH comprised between 4 and 9.

11. A method for obtaining a cosmetic product from a tiger nut milk concentrate according to claim 10, **characterized in that** the time of step c) is 20 minutes.

12. A method for obtaining a cosmetic product from a tiger nut milk concentrate according to claim 1, **characterized in that** said step d) for ultrasounds application lasts between 20 and 60 minutes.

13. A tiger nut milk base concentrate for preparing a cosmetic product, **characterized in that** said base concentrate comprises:
- tiger nut milk 100%
- natural or synthetic cosmetic ingredients or mixtures,
- distilled/deionized/thermal water
- natural or synthetic surfactants or mixtures and
- molasses alcohol.

14. A tiger nut milk base concentrate for preparing a cosmetic product, according to claim 13, **characterized in that** it comprises:
- tiger nut milk 100%
- natural or synthetic cosmetic ingredients or mixtures,
- distilled/deionized/thermal water
- natural or synthetic surfactants or mixtures and
- molasses alcohol, and
wherein said cosmetic ingredients are selected from combinations of two or more of the following:
- Anciano and Hamamelis distilled water;
- Sclerotium gum;
- water and Cyathea medullaris extract;
- betaine, water, glycerin, hydrogenated Lecithum, honey and pectin;
- ivy and myrrh extracts, leucine, valine, arginine and lysine;
- glycerin, water, lysolecithin, and seed oil from Perilla frutescens;
- corn and soy unsaponifiable
- propolis in hydroalcoholic solution,
- raspberry seeds virgin vegetable oil;
- rosehip virgin vegetable oil;
- Argan virgin vegetable oil;
- apricot nut virgin vegetable oil;
- tea tree oil.

15. A tiger nut milk base concentrate for preparing a cosmetic product, according to claim 13, **characterized in that** it comprises
- concentrate/traditional liquid tiger nut milk 100%: 30-50%
- thermal/deionized water: 5-20%
- natural and synthetic surfactants: 1-15%
- Anciano and Hamamelis distilled water: 1-10%
- Sclerotium gum: 1-4%
- water and Cyathea medullaris extract: 1-3%
- betaine, water, glycerin, hydrogenated Lecithum, honey and pectin: 3-5%
- ivy and myrrh extracts, Leucine, Valine, arginine and Lysine: 2-6%
- glycerin, water, lysolecithin, and seed oil from Perilla frutescens: 1-4%
- Corn and soy unsaponifiable: 2-5%
- propolis in hydroalcoholic solution: 1-8%
- raspberry seeds virgin vegetable oil;
- rosehip virgin vegetable oil;
- Argan virgin vegetable oil;
- apricot nut virgin vegetable oil and
- tea tree oil.

16. A cosmetic product **characterized in that** it is obtained by the method described in claims 1 to 12 and **in that** it comprises the tiger nut milk concentrate defined in any one of claims 13 to 15.

17. A cosmetic product according to claim 16, **characterized in that** it comprises:
- tiger nut milk base concentrate,
- emulsifier,
- active ingredients,
- essential oils,
- antioxidants,
- preservatives,
- natural dyes,
- perfumes, and
- sunscreens and sun protectors.

18. A cosmetic product according to claim 16 or 17, **characterized in that** it comprises a base concentrate having the following composition:
- concentrate/traditional liquid tiger nut milk (100%);
- thermal/deionized water;
- natural and synthetic surfactants,
- Anciano and Hamamelis distilled water;
- Sclerotium gum;
- water and Cyathea medullaris extract;
- betaine, water, glycerin, hydrogenated Lecithum, honey and pectin;
- ivy and myrrh extracts, leucine, valine, arginine and lysine;
- glycerin, water, lysolecithin, and seed oil from Perilla frutescens;
- Corn and soy unsaponifiable
- propolis in hydroalcoholic solution,
- raspberry seeds virgin vegetable oil;
- rosehip virgin vegetable oil; Argan virgin vegetable oil;
- apricot nut virgin vegetable oil;
- tea tree oil.

19. A cosmetic product according to one of claims 16 to 18, **characterized in that** it comprises a base concentrate having the following composition:
- concentrate/traditional liquid tiger nut milk (100%); 30-50%
- thermal/deionized water; 5-20%
- natural and synthetic surfactants, 1-15%
- Anciano and Hamamelis distilled water; 1-10%
- Sclerotium gum 1-4%
- water and Cyathea medullaris extract; 1-3%
- betaine, water, glycerin, hydrogenated Lecithum, honey and pectin 3-5%
- ivy and myrrh extracts, Leucine, Valine, arginine and Lysine; 2-6%
- glycerin, water, lysolecithin, and seed oil from Perilla frutescens: 1-4%
- Corn and soy unsaponifiable: 2-5%
- propolis in hydroalcoholic solution: 1-8%
- raspberry seeds virgin vegetable oil;
- rosehip virgin vegetable oil;
- Argan virgin vegetable oil;
- apricot nut virgin vegetable oil and
- tea tree oil.

20. A cosmetic product according to claim 16, **characterized in that** it is selected from creams, skin lotions, a facial cosmetic product, a color cosmetic product, a soap, bath gel, deodorants, a capillary cosmetic product, a toilet soap, fragrances, perfumes, colognes and products for sun protection.
